Europäisches Patentamt

**European Patent Office** (11) Publication number: **0 030 226**

Office européen des brevets **B1**

(12) **EUROPEAN PATENT SPECIFICATION**

(45) Date of publication of patent specification: **12.03.86** (51) Int. Cl.⁴: **A 01 N 59/06**

(21) Application number: **81100302.9**

(22) Date of filing: **02.11.78**

(60) Publication number of the earlier application in accordance with Art. 76 EPC: **0 002 003**

(54) **A composition containing a thixotropic aluminium derivative for use in agricultural application, a process for producing it and a process for the protection of agricultural products and agricultural products obtained thereby.**

(30) Priority: **12.11.77 GB 4717077**

(43) Date of publication of application: **10.06.81 Bulletin 81/23**

(45) Publication of the grant of the patent: **12.03.86 Bulletin 86/11**

(84) Designated Contracting States: **BE CH DE FR LU NL SE**

(56) References cited:
FR-A- 738 051
FR-A- 881 367
FR-A-2 274 278
GB-A- 572 142
GB-A- 784 706
GB-A-1 335 358
GB-A-1 460 328
US-A-3 873 686
US-A-4 034 067

R. TERTIAN, Bull. Soc. Chim., France 1953,99 and 1958,1301; Chem. Abs. 55 81279 (USSR Patent 132624,

(73) Proprietor: **Kuy, Bert**
**Hotzestrasse 28**
**CH-8006 Zürich (CH)**

(72) Inventor: **Kuy, Bert**
**Hotzestrasse 28**
**CH-8006 Zürich (CH)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to novel compositions of matter for use in agriculture.

The novel compositions of matter provide, when used in agricultural applications, a protective effect against fungal and bacterial afflictions of packaging materials and also for the protection of certain food-stuffs, fruit and vegetables against afflictions of fungi and bacteria, reduce the loss of water.

The active ingredient of the compositions of matter according to the present invention is a colloidal system of an aluminum chloride oxide hydrate having an atomic ratio of aluminum to chlorine in the range of from 5 to 1 to 6 to 1, and this can be presented by the general formula $Al_x(OH)_yCl_z$ wherein x, y and z are as required for the respective valencies of aluminum, chlorine and the hydroxy groups. It is believed that the more exact formula of the active ingredient is $[Al(OH)_3]_xAl(OH)_2Cl$ wherein x varies between 4 and 5. In the following the active material is designated as "aluminum hydroxide chloride".

The pH of the thixotropic composition varies between about 3.5 and about 5, and generally between 3.5 and 3.8.

The novel composition may optionally contain additional ingredients according to the specific uses of the said compositions. Said additional ingredients may be, for example, fatty substances such as lanolin, collagenase, polyalkylene, polyethyleneglycol and organic acid like tartaric, propionic, citric and lactic acid etc., of a germicidal, fungicidal, bacteriostatic or fungistatic substance and of glycerol. Examples are disinfectants, bacteriostats, bactericides and antimycotics.

This invention relates also to the process for producing a material for use in agricultural applications which comprises applying to the carrier a suitable quantity of the active ingredient of claim 1 so as to bring about a substantially homogeneous penetration of the active ingredient into the carrier.

The said thixotropic aluminum hydroxychloride is advantageously used to imbue a suitable inert carrier, such as a cellulosic material (paper, cellulose, cotton wool, cotton, etc.) or any other suitable carrier such as foams, fabrics, mixed fabrics, non-woven fabrics, gauze and the like more.

A suitable quantity of the active ingredient is used to imbue or impregnate the said carrier, the quantity of the active ingredient being such as to provide as high an effective quantity of the active material as can be supported by said carrier. Representative values of such quantities are from 10 to 35 g per 100 cm² of a material such as plastic foam or gauze.

When admixed with a suitable quantity of water, there is obtained a thixotropic system into which there are readily incorporated substances such as lanolin; which lather is generally obtained as stable system with aqueous ingredients only in conjunction with other constituents such as surface active agents or the like.

The active ingredient is a micro-crystalline aluminum hydroxide in which part of the hydroxy groups have been replaced by chlorine, to give the above defined composition with the stipulated molar ratio of aluminum to chlorine. The substance in water, is thixotropic: about 10% by weight of the said hydroxy-chloride in water forms a gel which can be easily liquified by application of mechanical forces, such as by shaking, brushing or the like. The viscosity decreases thereby about 100 fold, from values in the range of 10,000 cps to about 100 cps. When left to stand it reverts again to the initial viscosity.

When the thixotropic gel is used in conjunction with a suitable carrier, the said carrier is imbued with the gel either by pressure; under reduced pressure, by brushing, coating or spraying. There is obtained a substantially homogenous product carrying a predetermined quantity of active material per unit area. .

The thixotropic colloid aluminum hydroxide chloride can be used in agriculture for the protection against afflictions by various pests, and especially of fungi. Extensive tests have shown that the thixotropic material can be applied directly to various types of agricultural products such as grains, maize, barley, and the like, to various types of fruit etc. There is formed a thin coating which adheres well to the surface of the treated agricultural product and this thin film constitutes a protective barrier against various afflictions, and especially against fungal attack. The prevention of fungal growth prevents the production of aflotoxins which are presently considered to be a serious health hazard. When subjected to rain or to irrigation, the protective coating is not readily washed off and it retains its efficacy over prolonged periods of time. Laboratory tests were carried out with various types of fruit, such as strawberries, peaches, citrus fruit, cherries, grapes, pears and apples. Tests were carried out with vegetables such as cucumbers, egg plants, potatoes and tomatoes. These were sprayed or brushed with a colloidal suspension of the thixotropic aluminum hydroxy chloride containing a small quantity of a non-ionic surfactant. The resulting non-poisonous and harmless coating substantially reduced fungal attack and also substantially reduced oxidative effects. Formation of spots was retarded and evaporation of water was substantially reduced. For example, citrus fruit retained their fresh appearance and lost weight at a substantially reduced rate compared with similar untreated fruit.

Meat and meat products, such as meat, sausages, smoked meat and the like can be protected against fungal attack and against weight loss by a thin protective coating of this type. Generally there were used colloidal thixotropic compositions containing from about 3 to 10 per cent by weight of the aluminum hydroxy chloride compound.

Tests were carried out with corn and with maize and laboratory tests prove a substantial reduction of fungal affliction of the treated samples. Furthermore, it was found that the shrinkage of the treated corn is reduced and there is a decreased formation of dust. There is obtained a substantially uniform surface film which shows a good adherence and which is very little affected by irrigation or by rain. The active ingredient is characterized by its very low solubility, and there is practically no diffusion into the treated product. Due to its nature, the active ingredient is very stable.

The novel composition can be mechanically applied by rolling, brushing or spraying but also by dipping in any quantity required on or in the suitable carrier.

The novel composition can be distributed easily in minimum or maximum quantities in an equal and homogeneous manner without flowing off and has a strong adhesion on porous, homogeneous or lipoid surfaces of materials like textiles, synthetic and natural materials.

For addition of further additives to the novel composition there is no need to use an emulsifier.

The novel composition is stable over a long period of time. It can be brought into any consistence required by applying pressure or any mechanical forces like vacuum. This is very important for its economic use in agriculture.

The new composition is reversible in binding high volumes of moisture or water. It is insoluble in water and difficultly washed off. Its osmotic properties remain unchanged and are amongst the strongest among the known aluminum compounds.

The novel composition shows a long time activity at low dosages. The novel composition has a reversible adhesive power and therefore reactivates itself continuously effectively. By its insolubility there results an increased effect, no toxidity and therefore a controlled application.

Other and further features of the invention will become apparent hereinafter from the following detailed description, which is to be construed in a non-limitative manner.

### Example 1

A thixotropic substance is prepared from colloidal aluminum-hydroxide containing about 5 to 1 to about 6 to 1 atoms of aluminum per atom of chlorine in a concentration of about 10 weight per cent in water. The pH of the substance is about 4.0 and when at rest the viscosity is about 10.000 cps. Upon vigorous shaking the viscosity goes down to about 100 cps.

### Example 2

A thixotropic composition was prepared by incorporating 5 grams of lanolin into a thixotropic composition of Example 1. The lanolin was gradually introduced with vigorous agitation and there was added a quantity of about 0.3 g potassium chloride per 100 mg of the thixotropic substance. A uniform, stable composition was obtained.

### Example 3

A thixotropic composition was prepared by incorporating 1 to 3 per cent by weight of PVP-iodine into a composition of Example 1. A germicidal substance was obtained.

### Example 4

A thixotropic substance was prepared by incorporating a quantity of from 1 to 5 per cent of alginate into a composition of Example 1.

### Example 5

A composition of matter was prepared by incorporating up to about 70% by weight of pulverized polymeric foam (such as polyurethane powder) into a liquified composition according to claim 1. There was obtained a kneadable material which solidifies under refrigeration or upon evaporation of the larger part of its water content.

### Example 6

A highly porous polymeric foam (polyurethane foam) in ribbon form was imbued by brushing with liquified material prepared according to Example 1. The quantity used was about 30 g of the said substances per 100 cm$^2$ of the said carrier. By application of pressure on the foam carrying the thixotropic material a substantially uniform distribution of the said active ingredient, throughout the carrier was obtained when the pressure was released after the compression of the said foam. This foam can be refrigerated and used for the effective application of cold during a prolonged period of time.

### Example 7

A polyurethane ribbon of the type used in Example 6 was placed on a thin non-woven fabric and impregnated as set out in Example 6 by applying the thixotropic material from the open side of the foam.

### Example 8

Gauze was prepared so as to provide about 20 grams of the substance of Example 1 per 100 gram of the gauze. The impregnation was effected by impregnation or by brushing.

### Example 9

Non woven cellulose fabric was imbued in a manner set out in Example 7. The quantity used was 10 gram of the material of Example 1 per 100 gram of the fabric.

### Example 10

A composition according to Example 1 was tested in order to evaluate its activity on fruit and vegetables. A peach was cut in half, and one half was treated by the application of a thin layer of such composition. After 4 hours the treated surface was clean and free of discoloration. The other, untreated half exhibited brownish discolored areas. After a further 10 hours the treated area was still clear, while the untreated one was covered with fungal growth. The two halves were placed in contact with each other and left overnight. The untreated one exhibited further deterioration while the treated one remained clear.

A further test was carried out with another peach. The treated half remained free of fungal attack after 48 hours while the untreated half was badly deteriorated.

A peach was coated with a thin layer of the composition of Example 1 and placed amongst already slightly contaminated peaches. After one week the untreated fruit were completely covered with fungi whereas the treated was quite clean.

**Claims**

1. A colloidal composition of matter for use in agriculture comprising as active ingredient an aluminum hydroxy chloride which is a thixotropic microcrystalline aluminum chloride oxide hydrate having an atomic ratio of aluminum to chlorine of from 5 to 1 to 6 to 1, said colloid containing said active material in a concentration of about 3 to 20 percent by weight.

2. A material for use in agricultural applications, which comprises a composition according to claim 1 as active ingredient supported by a suitable carrier.

3. A material according to claim 2 wherein the carrier is a foam or a cellulosic material.

4. A material according to claim 3, wherein the carrier is a foam, paper, gauze, cotton-wool or non-woven cellulosic fabric.

5. A composition according to claim 1, containing a quantity of fatty substance, collagenase, polyalkylene, polyethyleneglycol and organic acid, of germicidal, fungicidal, bacteriostatic or fungistatic substance and of glycerol.

6. A composition for use in agriculture as protective agent against plant pests and especially against fungal attack of plants, fruit, grains and vegetables comprising as active ingredient a composition as claimed in claim 1.

7. A process for producing a material according to any of claims 2 to 4 which comprises applying to the carrier a suitable quantity of the active ingredient of claim 1 so as to bring about a substantially homogeneous penetration of the active ingredient into the carrier.

8. A process according to claim 7, wherein said carrier is imbued with the composition of claim 1 either by pressure or under reduced pressure.

9. A process for the protection of agricultural products which comprises applying the active ingredient according to claim 1 by spraying, dipping or brushing.

10. Agricultural products, whenever provided with a protective coating containing the active material of claim 1.

**Patentansprüche**

1. Kolloidale Zusammensetzung zur Verwendung in der Landwirtschaft, dadurch gekennzeichnet, daß sie als aktiven Bestandteil ein Aluminiumhydroxychlorid enthält, das ein thixotropes mikrokristallines Aluminium-chloridoxidhydrat mit einem Atomverhältnis von Aluminium zu Chlor von 5:1 bis 6:1 ist, wobei das Kolloid das aktive Material in einer Konzentration von etwa 3 bis 20 Gew.-% enthält.

2. Material zur Verwendung bei landwirtschaftlichen Anwendungen, dadurch gekennzeichnet, daß es eine Zusammensetzung nach Anspruch 1 als aktiven Bestandteil aufweist, wobei die Zusammensetzung von einem geeigneten Träger getragen wird.

3. Material nach Anspruch 2, dadurch gekennzeichnet, daß der Träger ein Schaum oder ein cellulosehaltiges Material ist.

4. Material nach Anspruch 3, dadurch gekennzeichnet, daß der Träger Schaum bzw. Schaumstoff, Papier, Gaze, Watte oder ein nichtgewebtes cellulosehaltiges Flächenmaterial ist.

5. Zusammensetzung nach Anspruch 1, dadurch gekennzeichnet, daß sie eine Menge an fettartiger Substanz, Collagenase, Polyalkylen, Polyethylenglykol und organischer Säure einer germiziden, fungiziden, bakteriostatischen oder fungistatischen Substanz und Glycerin enthält.

6. Zusammensetzung zur Verwendung in der Landwirtschaft als Schutzmittel gegen Pflanzenschädlinge und insbesondere gegen Pilzbefall bei Pflanzen, Obst, Körnern und Gemüse, dadurch gekennzeichnet, daß sie als aktiven Bestandteil eine Zusammensetzung nach Anspruch 1 enthält.

7. Verfahren zur Herstellung eines Materials nach einem der Ansprüche 2 bis 4, dadurch gekennzeichnet, daß man eine geeignete Menge des aktiven Bestandteils nach Anspruch 1 auf einen Träger bringt, so daß eine im wesentlichen homogene Penetration des aktiven Bestandteils in den Träger erfolgt.

8. Verfahren nach Anspruch 7, dadurch gekennzeichnet, daß der Träger mit der Zusammensetzung nach Anspruch 1 entweder durch Druck oder unter verringertem Druck getränkt wird.

9. Verfahren zum Schutz von landwirtschaftlichen Produkten, dadurch gekennzeichnet, daß man den aktiven Bestandteil nach Anspruch 1 durch Sprühen, Eintauchen oder Aufbürsten aufbringt.

10. Landwirtschaftliche Produkte, dadurch gekennzeichnet, daß sie einen Schutzüberzug aufweisen, der das aktive Material nach Anspruch 1 enthält.

**Revendications**

1. Composition colloidale utilisable dans le domaine d'agriculture, caractérisée en ce qu'elle comprend à titre d'ingrédient actif un gel thixotrope aqueux d'environ 3 à 20% en poids d'hydroxychlorure d'aluminium microcristallin ayant un rappor atomique d'aluminium à chlore allant de 5:1 à 6:1.

2. Un matériel utilisable en applications agricoles, caractérisé par une composition selon revendication no. 1 contenant un ingrédient actif supporté par un support adepte.

3. Un matériel selon la revendication no. 2 où le support est constitué par une mousse ou du matériel à base de cellulose.

4. Un matériel selon la revendication no. 3 où le support est de mousse, papier, gaze, cotton ou tissu cellulosique non tissé.

5. Une composition selon revendication 1, contenant une certaine quantité de substance grasse, de collagénase, de polyalkylène, de polyéthylèneglycol, d'acide organique d'une substance germicide, fongicide, bactériostatique ou fongistatique ou del glycérol.

6. Une composition utilisable dans le domaine agricole comme agent de protection contre attaques fongicides aux plantes, fruits, grains et légumes, comprenant un ingrédient actif d'une composition telle que revendique sous no. 1.

7. Un procédé pour produire un matériel selon les revendications 2 à 4 ce qui comprend l'application d'une quantité adepte de l'ingrédient actif selon revendication no. 1 dans un support, afin d'achever une pénétration homogène de l'ingrédient actif dans le support.

8. Un procédé selon revendication no. 7 où le support mentionné est trempé par la composition de la revendication 1 soit par pression ou sous pression réduite.

9. Un procédé pour la protection de produits agricoles ce qui comprend l'application de l'ingrédient actif selon la revendication 1 en évaporisateur, trempage ou brossage.

10. Produits agricoles, quand pourvus d'une couche protectrice contenant le matériel actif de la revendication no. 1.